(19) Europäisches Patentamt
European Patent Office
Office européen des brevets

(11) Publication number: **0 061 268**
**B1**

(12) **EUROPEAN PATENT SPECIFICATION**

(45) Date of publication of patent specification: **28.11.84**

(51) Int. Cl.³: **C 07 C 179/15,** C 07 C 178/00

(21) Application number: **82301266.1**

(22) Date of filing: **12.03.82**

(54) **Stabilised peroxide composition and method of producing the same.**

(30) Priority: **14.03.81 GB 8108084**

(43) Date of publication of application:
**29.09.82 Bulletin 82/39**

(45) Publication of the grant of the patent:
**28.11.84 Bulletin 84/48**

(84) Designated Contracting States:
**AT BE CH DE FR IT LI LU NL SE**

(56) References cited:
**BE-A- 560 389**
**GB-A-1 085 787**
**US-A-3 770 816**

(73) Proprietor: **STERWIN AG.**
**Zeughausgasse 9**
**CH-6300 Zug (CH)**

(72) Inventor: **Brocklehurst, Peter**
**87 Cowley Lane**
**Chapeltown Sheffield S30 4SX (GB)**
Inventor: **Ferguson, Angus Scott**
**212 Thompson Hill**
**High Green Sheffield S30 4JU (GB)**

(74) Representative: **Green, Alan James et al**
**Sanderson & Co. 97 High Street**
**Colchester Essex C01 1TH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 061 268 B1

## Description

The present invention relates to a stabilised peroxide composition, in particular a composition comprising succinyl peroxide.

Succinyl peroxide, which is also known as disuccinyl peroxide or succinic acid peroxide is a compound of the formula:

$$(HOOC.CH_2.CH_2.CO)_2O_2$$

Succinyl peroxide is known for use as a polymerisation initiator, as a deodorant and as an antiseptic. In the past the compound has been sold in the dry powder form for use primarily as a polymerisation initiator, but it was found that the dry powder is friction sensitive and, under certain circumstances, could explode. In order to overcome this problem succinyl peroxide is now marketed as a 70 per cent by weight paste in water.

However, succinyl peroxide as a paste in water is unstable at ambient temperatures, the peroxide decomposing by hydrolysis, and stability can only be ensured by storing the paste under refrigerated conditions, typically at −10°C. It will be appreciated that the need to store and transport the paste under refrigerated conditions is a significant disadvantage, especially when the peroxide is required for use in applications where say relatively small unit doses of peroxide are to be stored before use, for example as an antiseptic.

We have now found surprisingly that an intimate mixture of succinyl peroxide and a water-soluble dehydrating agent which does not react with succinyl peroxide provides a composition which is stable on storage at ambient temperatures and which does not possess the friction sensitivity of dry, powdered succinyl peroxide *per se*.

Accordingly, the present invention provides a stabilised succinyl peroxide composition, characterised in that the succinyl peroxide is admixed with a water-soluble mineral salt stabilising agent which can function as a dehydrating agent but which does not react with succinyl peroxide.

In the compositions of the invention the stabilising agent must be one that is water soluble and one that is sufficiently inert not to react with succinyl peroxide. The stabilising agent must also be capable of functioning as a dehydrating agent, and in particular, is preferably a dehydrating agent which can or does include water bound as water of crystallisation. Moreover, since the creation of a high pH could cause the succinyl peroxide to decompose, additionally it is preferred that the stabilising agent should generally be one capable of providing an acid pH in solution, preferably a pH of below 5, for example, about 4 to about 2.5. Most preferably the stabilising agent does not materially alter the pH of succinyl peroxide in solution.

Preferred dehydrating agents are inorganic compounds which can exist as partial hydrates and especially preferred dehydrating agents are sodium and magnesium sulphates, which may be used in anhydrous form or more preferably in a partially hydrated form. Of these dehydrating agents magnesium sulphate is the most preferred, in particular as $MgSO_4.nH_2O$, wherein n has a value of from about 2 to less than 7, preferably about 2 to about 3.

In the compositions of the invention it is preferred that for every 1 part by weight of succinyl peroxide the composition should comprise from about 0.1 to about 12.5 parts by weight of bound water and from about 1 to about 50, preferably from about 1 to about 25, parts by weight of a dehydrating agent. More preferably, the proportion of bound water is from about 0.4 to about 1.0 parts by weight and the dehydrating agent is present in a proportion of from about 1.0 to about 3.5 parts by weight, and most preferably the proportion of bound water is about 0.4 to about 0.7 parts by weight and the dehydrating agent is present in a proportion of from about 1.1 to about 2.2 parts by weight.

The compositions of the invention suitably may be prepared by mixing a dehydrating agent with an aqueous paste of succinyl peroxide, typically the 70% by weight paste now commercially available.

Accordingly, the invention also provides a method of producing succinyl peroxide in a stable particulate solid form whereby it may be stored at ambient temperature with minimum decomposition, which method comprises treating a paste of succinyl peroxide in water with a water-soluble dehydrating agent which does not react with succinyl peroxide, the dehydrating agent being employed in an amount sufficient to provide a dry particulate solid mixture, preferably a dry powder.

In the method of the invention the aqueous paste of succinyl peroxide typically will be the about 70% by weight paste in water which is commercially available, although pastes having a peroxide content generally down to about 40% by weight, typically down to about 50% by weight, may be employed, and the upper limit of peroxide may, if desired, be up to about 90%. While the content of the paste will usually depend on the most convenient manufacturing source available in any particular instance, whatever the content of the paste used it can be treated to provide a dry mixture by mixing generally with from about 40 to about 95% by weight of dehydrating agent, preferably a sodium or magnesium sulphate as described above, based on the total weight of dehydrating agent and the paste. Preferably from about 45 to about 80% by weight of dehydrating agent is employed based on the total weight of dehydrating agent and paste.

Where the dehydrating agent employed is a partially hydrated material it is to be understood that the amount employed may have to be increased in proportion to the degree of hydration of the agent. Also, since the dehydrating agent acts to remove water from the paste the level of hydration (if any) in the dehydrating agent will initially be below that mentioned above in connection with the compositions of the invention. Thus, in the case of hydrates of magnesium sulphate, in the formula $MgSO_4.n'H_2O$ the value of n' will be less than the corresponding values for n mentioned above, and typically n' will have a value of from about 1 to about 4, preferably from about 1 to about 2.

Preferably, the dehydrating agent is employed as a fine powder, and the treatment may be effected by simple mixing for a suitable time to provide a dry mixture. Generally a mixing time of from about 15 minutes to about 6 hours will be sufficient, and typically a mixing time of from about 30 minutes to about 4 hours may be employed.

In the method of the invention, the amount of dehydrating agent and the mixing time generally are adjusted so that there is produced an intimate mixture of ingredients in particulate solid form which gives the appearance of a dry solid, for example, a dry powder. Such a dry solid is a dry mixture of the kind required herein whether or not the mixture as a whole is dry in any absolute sense. Thus, provided any water present is captured by dehydrating agent and is not free to wet the peroxide, the mixture is dry.

In preparing the compositions of the invention other optional ingredients may be included as and when desired. Thus, the compositions may include one or more detergent materials, chelating agents, hydrogen peroxide donors, e.g. urea hydrogen peroxide adduct or sodium perborate, and colouring agents. For example, the compositions may include from about 1% to about 2% by weight of a solid anionic, e.g. sodium lauryl sulphate, or non-ionic detergent, and colour may be provided using either a dyestuff or a pigment, e.g. ultra-marine. In particular, the compositions preferably may include a dyestuff (typically one giving a blue colour) which is stable in the presence of succinyl peroxide when in aqueous solution at dilutions such as those mentioned below. As an example of such a dyestuff there may be mentioned the copper phthalocyanine complex C.I. Acid Blue 254.

The compositions of the present invention are biocidal, and accordingly are useful as cleaning and/or disinfecting and/or sterilising and/or antiseptic compositions. The compositions by virtue of the fact that they contain succinyl peroxide are not only bactericidal but also fungicidal, sporicidal and perhaps viricidal.

In particular, the compositions of the invention may be used as general purpose hard surface cleaners, as instrument sterilisers, or as a replacement for Iodophor. Moreover, because the compositions are in a stable dry powder form, they may be packaged for distribution, storage and use without any special restrictions being imposed on transport and storage conditions. As shown below the compositions remain in an active condition even after a storage period well in excess of one year.

When required for use the compositions are dissolved in water at a high dilution, typically at a dilution of from 0.05% to 2% w/w (based on a succinyl peroxide content of about 20% by weight) and used immediately after they are made up in diluted form.

Following is a description by way of example of the preparation of stabilised peroxide compositions in accordance with the invention.

Example 1

0.2 kg of a 70% by weight succinyl peroxide paste in water obtained from Laporte Industries were mixed with various proportions of sodium and magnesium sulphate to obtain a dry mixture. The dehydrating agent employed, its amount and the mixing conditions and time are shown in Table 1 below:

TABLE 1

| % w/w Succinyl peroxide as a 70% w/w paste in water | % w/w Magnesium sulphate monohydrate | % w/w Sodium sulphate-anhydrous | Mixing conditions | Mixing time |
|---|---|---|---|---|
| 10 | 90 | — | Tumble mixer | 4 hours |
| 20 | 80 | — | Tumble mixer | 4 hours |
| 30 | 70 | — | Tumble mixer | 4 hours |
| 50 | 50 | — | Tumble mixer | 4 hours |
| 10 | — | 90 | Tumble mixer | 4 hours |
| 20 | — | 80 | Tumble mixer | 4 hours |
| 30 | — | 70 | Tumble mixer | 4 hours |

Under storage conditions at ambient temperature each of the above-prepared dry mixtures gave results which indicated that a shelf life in excess of one year can be obtained in accordance with the present invention. In this connection, and by way of example, reference is made to the accompanying drawing which shows a graph of activity vs. time for a 70% by weight succinyl peroxide paste in water compared with two of the dry mixtures prepared as described above. The results expressed graphically were obtained from batches stored at about 20°C, the % w/w of succinyl peroxide being determined by sampling at suitable intervals and estimating the remaining succinyl peroxide by standard iodometric methods.

In the drawing, curve A shows the activity of the 70% paste, from which it can be seen that activity decreases to a low level within about 60 days and at about 90 days the activity is so low as to be virtually not measurable. By contrast line B (mixture of 30% w/w of 70% paste with 70% w/w magnesium sulphate monohydrate) and line C (mixture of 30% w/w of 70% paste with 70% w/w anhydrous sodium sulphate) show that in accordance with the present invention activity is maintained at a significant level over a much longer period of time.

Example 2

50 parts by weight of a 70% by weight succinyl peroxide paste in water obtained from Laporte Industries were mixed with 50 parts by weight of magnesium sulphate monohydrate to obtain a dry mixture. The initial succinyl peroxide assay given by the paste was 33.3% w/w and after 14 months of storage at ambient temperature the mixture gave a succinyl peroxide assay of 31.6% w/w.

Example 3

A large scale batch of 50 kg of a stabilised peroxide composition was prepared by mixing 20 kg of a 70% by weight of succinyl peroxide paste in water with 30 kg of magnesium sulphate dihydrate to form a mixture. The dry mixture was then packed as 5 kg lots in polyethylene bags and those in turn were stored in a fibre board drum.

The initial succinyl peroxide assay given by the paste was 29.0% w/w. After mixing and storage at ambient temperature for one month there was an initial drop in the assay to a figure of 27.0% w/w, but after a total of 5 months' storage at ambient temperature the succinyl peroxide assay remained at 27.0% w/w.

**Claims**

1. A stabilised succinyl peroxide composition characterised in that the succinyl peroxide is admixed with a water-soluble mineral salt stabilising agent which can function as a dehydrating agent but which does not react with succinyl peroxide.

2. A composition according to claim 1, wherein the stabilising agent is an inorganic compound in a partially hydrated form which gives an acid pH in solution preferably of below 5, and preferably is a partial hydrate of sodium or magnesium sulphate.

3. A composition according to claim 2, wherein the stabilising agent is $MgSO_4.nH_2O$, where n has a value of from about 2 to less than 7, preferably of from about 2 to about 3.

4. A composition according to any one of the preceding claims which for every 1 part by weight of succinyl peroxide comprises from about 0.1 to about 12.5 parts by weight of bound water and from about 1 to about 50 parts by weight of a dehydrating agent, preferably from about 0.4 to about 1.0 parts by weight of bound water and from about 1.0 to about 3.5 parts by weight of dehydrating agent, more preferably about 0.4 to about 0.7 parts by weight of bound water and from about 1.1 to about 2.2 parts by weight of dehydrating agent.

5. A composition according to any one of the preceding claims, which also includes one or more detergent materials, chelating agents, hydrogen peroxide donors and colouring agents.

6. A composition according to claim 5, which includes from about 1% to about 2% by weight of a solid anionic or non-ionic detergent and/or a dyestuff stable in the presence of succinyl peroxide in dilute aqueous solution.

7. A method of producing succinyl peroxide in a stable particulate solid form whereby it may be stored at ambient temperature with minimum decomposition, which method comprises treating a paste of succinyl peroxide in water with a water-soluble dehydrating agent which does not react with succinyl peroxide, the dehydrating agent being employed in an amount sufficient to provide a dry particulate solid mixture, preferably a powder.

8. A method according to claim 7, wherein the paste comprises at least about 40% by weight of succinyl peroxide, the balance being water, and preferably about 70% by weight of succinyl peroxide and about 30% by weight of water.

9. A method according to claim 7 or claim 8, wherein from about 40% to about 95% by weight of dehydrating agent is employed based on the total weight of dehydrating agent and paste, and preferably from about 45% to about 80% by weight of dehydrating agent.

10. A method according to any one of claims 7 to 9, wherein the dehydrating agent is anhydrous sodium or magnesium sulphate or a partial hydrate thereof, preferably magnesium sulphate monohydrate or dihydrate.

**Patentansprüche**

1. Stabilisierte Succinylperoxidzusammensetzung, dadurch gekennzeichnet, daß das

Succinylperoxid mit einem Wasserlöslichen Mineralsalzstabilierungsmittel vermischt ist, das als Dehydratisierungsmittel wirken kann, aber nicht mit dem Succinylperoxid reagiert.

2. Zusammensetzung nach Anspruch 1, dadurch gekennzeichnet, daß das Stabilisierungsmittel eine anorganische Verbindung in einer partiell hydratisierten Form, die in Lösung einen pH-Wert von vorzugsweise unter 5 liefert, und vorzugsweise ein partielles Hydrat von Natrium- oder Magnesiumsulfat ist.

3. Zusammensetzung nach Anspruch 2, dadurch gekennzeichnet, daß das Stabilisierungsmittel $MgSO_4.nH_2O$ ist, wobei n einen Wert von etwa 2 bis weniger als 7 und vorzugsweise von etwa 2 bis etwa 3 besitzt.

4. Zusammensetzung nach einem der vorangegangenen Ansprüche, dadurch gekennzeichnet, daß sie auf ein Gewichtsteil Succinylperoxid etwa 0,1 bis etwa 12,5 Gewichtsteile gebundenes Wasser und etwa 1 bis etwa 50 Gewichtsteile eines Dehydratisierungsmittels, vorzugsweise etwa 0,4 bis 1,0 Gewichtsteile gebundenes Wasser und etwa 1,0 bis 3,5 Gewichtsteile Dehydratisierungsmittel und insbesondere etwa 0,4 bis etwa 0,7 Gewichtsteile gebundenes Wasser und etwa 1,1 bis etwa 2,2 Gewischtsteile Dehydratisierungsmittel enthält.

5. Zusammensetzung nach einem vorangegangenen Ansprüche, dadurch gekennzeichnet, daß sie außerdem ein oder mehrere Detergentien, Chelatisierungsmittel, Wasserstoffperoxiddonatoren und Färbehilfsmittel enthält.

6. Zusammensetzung nach Anspruch 5, dadurch gekennzeichnet, daß sie etwa 1 bis etwa 2 Gew. % eines festen anionischen oder nichtionischen Detergens und/oder eines Farbstoffes enthält, die Gegenwart von Succinylperoxid in verdünnter wässeriger Lösung stabil sind.

7. Verfahren zur Herstellung von Succinylperoxid in stabiler teilchenförmiger fester Form, so daß es bei Raumtemperatur unter minimaler Zersetzung gelagert werden kann, dadurch gekennzeichnet, daß eine Paste von Succinylperoxid in Wasse mit einem wasserlöslichen Dehydratisierungmittel, das nicht mit dem Succinylperoxid reagiert, behandelt wird, wobei das Dehydratisierungsmittel in einer ausreichenden Menge verwendet wird, um eine trockne teilchenförmige feste Mischung und vorzugsweise ein Pulver zu liefern.

8. Verfahren nach Anspruch 7, dadurch gekennzeichnet, daß die Paste mindestens etwa 40 Gew. % Succinylperoxid und im übrigen Wasser und vorzugsweise etwa 70% Gew. % Succinylperoxid und etwa 30 Gew. % Wasser enthält.

9. Verfahren nach Anspruch 7 oder 8, dadurch gekennzeichnet, daß bezogen auf das Gesamtgewicht von Dehydratisierungsmittel und Paste etwa 40 bis etwa 95% Gew. % Dehydratisierungsmittel und vorzugsweise etwa 45 bis etwa 80 Gew. % Dehydratisierungsmittel verwendet werden.

10. Verfahren nach einem der Ansprüche 7 bis 9, dadurch gekennzeichnet, daß das Dehydratisierungsmittel wasserfreies Natrium oder Magnesium-sulfat oder ein parielles Hydrat derselben, vorzugsweise Magnesium-sulfatmonohydrat oder -dihydrat ist.

## Revendications

1. Une composition stabilisée de peroxyde de succinyle, caractérisée en ce que le peroxyde de succinyle est ajouté et mélangé à un agent stabilisant d'un sel minéral soluble dans l'eau qui peut fonctionner comme un agent de déshydratation, mais qui ne réagit pas avec le peroxyde de succinyle.

2. Une composition selon la revendication 1, caractérisé en ce que l'agent stabilisant est une composé minéral dans une forme partiellement hydratée qui donne un pH acide en solution, de préférence inférieur à 5, et de préférence est un hydrate de sodium partiel ou du sulfate de magnésium.

3. Une composition selon la revendication 2, caractérisé en ce que l'agent stabilisant est $MgSO_4.nH_2O$, dans lequel n a une valeur d'environ 2 à moins de 7, de préférence d'environ 2 à 3 environ 3.

4. Une composition selon l'une quelconque des revendications précédentes qui, pour chaque partie en poids de peroxyde de succinyle, comprend d'environ 0,1 à environ 12,5 parties en poids d'eau liée et d'environ 1 à environ 50 parties en poids d'une agent déshydratant, de préférence d'environ 0,4 à 1,0 partie en poids d'eau liée et d'environ 1,0 à environ 3,5 parties en poids d'agent déshydrant, de préférence de 0,4 à 0,7 partie en poids d'eau liée et d'environ 1,1 à environ 2,2 parties en poids d'agent déshydratant.

5. Une composition selon l'une quelconque des revendication précédentes, qui comprend également un ou plusieurs agents détergents, agents chélatants, donneurs de peroxyde d'hydrogéne et agents colorants.

6. Une composition selon la revendication 5 qui comprend d'environ 1% environ 2% d'un solide anionique ou d'un détergent non ionique et/ou colorant stable en présence de peroxyde de succinyle en solution aqueuse dilufe.

7. Un procédé d'obtention de peroxyde de succinyle dans une forme solide particulaire stable, ce par quoi il peut être conservé à température ambiante avec le minimum de décomposition, lequel procédé comprend le traitement d'une pâte de peroxyde de succinyle dans l'eau avec un agent déshydratant soluble dans l'eau, qui ne réagit pas avec le peroxyde de succinyl, l'agent déshydratant étant utilisé en quantité suffisante pour donner un mélange solide particulaire sec, de préférence une poudre.

8. Un procédé selon la revendication 7, dans

lequel la pâte comprend au moins environ 40% en poids de peroxyde de succinyl, le complément étant l'eau, de préférence environ 70% de peroxyde de succinyle et environ 30% en poids d'eau.

9. Un procédé selon la revendication 7 ou 8, caractérisé en ce qu'environ 40 à environ 95% en poids d'agent déshydratant sont utilisés sur la base du poids total d l'agent déshydratant et de la pâte, et de préférence d'environ 45 à environ 80% en poids d'agent déshydratant.

10. Un procédé selon l'une quelconque des revendications 7 à 9 caractérisé en ce que l'agent déshydratant est du sodium anhydre ou du sulfate de magnésium ou un hydrate partiel de celui-ci, de préférence du monohydrate ou du dihydrate de sulfate de magnesium.

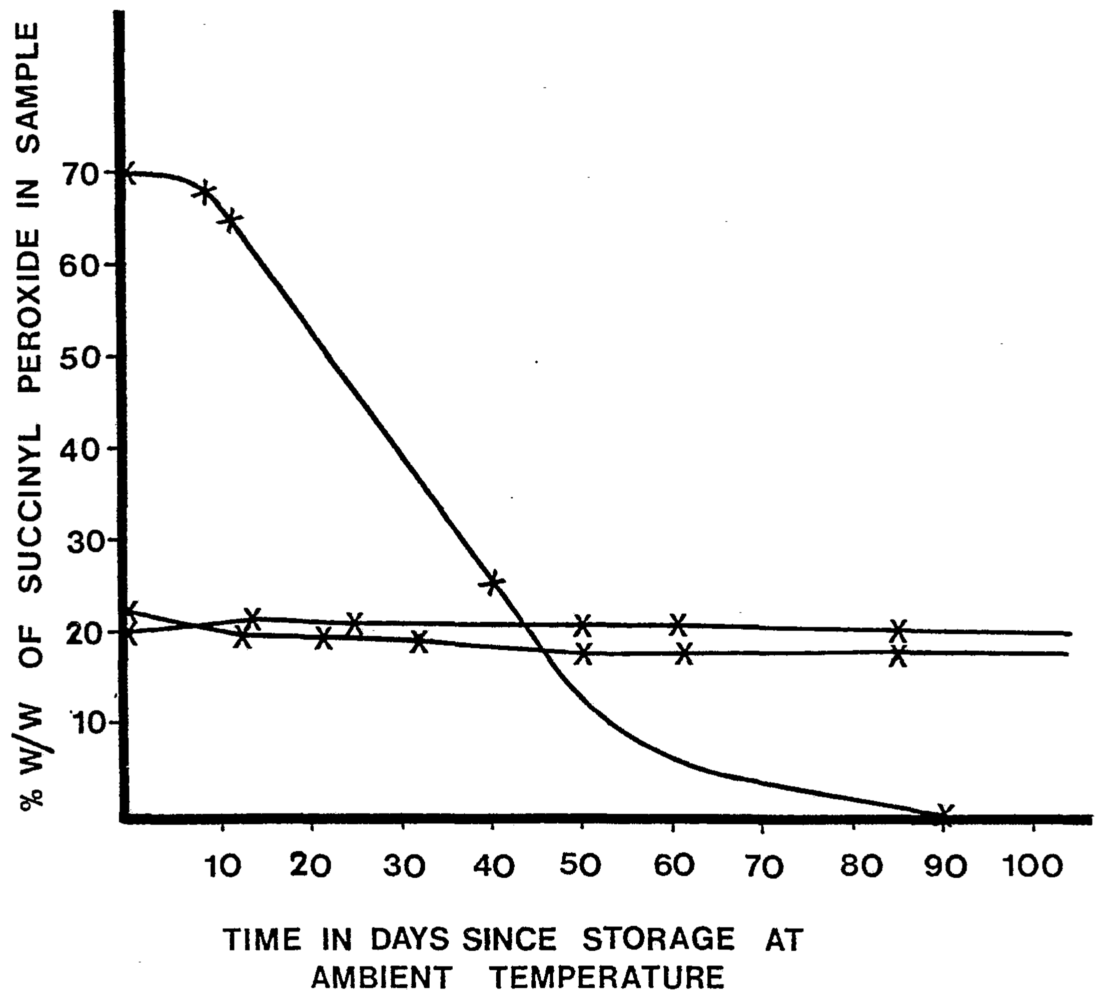
% W/W OF SUCCINYL PEROXIDE IN SAMPLE
70
60
50
40
30
20
10
10 20 30 40 50 60 70 80 90 100
TIME IN DAYS SINCE STORAGE AT
AMBIENT TEMPERATURE